# EUROPEAN PATENT APPLICATION

(11) **EP 4 595 881 A1**
(43) Date of publication of application: **06.08.2025**
(21) Application number: 25152643.0
(22) Date of filing: 17.01.2025
(51) Int. Cl.: A61B 5/145, A61B 5/00, A61B 5/01, G01N 27/414, A61B 10/00

(54) **VAGINAL APPARATUS**

(30) Priority: 30.01.2024 GB 202401204
(71) Applicant: Yon E Global Holding, 1017BR Amsterdam (NL)
(72) Inventor: VERWER, Roswitha, 1017BR Amsterdam (NL)
(74) Representative: HGF

(57) **Abstract**

Aspects relate to a vaginal device, a method of manufacture thereof, and a kit of parts. The vaginal device comprises: an elongated body comprising an insertion portion at a first end of the body, the insertion portion intended for insertion into a vagina; and a pH sensing device located within the elongated body, the pH sensing device comprising: a pH sensor, the pH sensor comprising a glass-free sensing surface located on a surface of the insertion portion, the glass-free sensing surface configured to contact a substance to be pH tested; and a controller connected to the pH sensor, the controller configured to receive pH sensing signalling from the pH sensor and output an indication of a pH of a substance contacted by the glass-free sensing surface. The vaginal device may comprise a temperature sensing device located within the elongated body, the temperature sensing device comprising: a temperature sensor located in the insertion portion and configured to sense a temperature of a substance in contact with the temperature sensor; and a temperature controller connected to the temperature sensor, the temperature controller configured to receive temperature sensing signalling from the temperature sensor and output an indication of a temperature sensed by the temperature sensor. Vaginal temperature and pH may thus both be measured simultaneously in such examples.

## Description

### TECHNICAL FIELD

The present disclosure relates to a vaginal device, a method of manufacture of a vaginal device, and a kit of parts including the vaginal device.

### BACKGROUND

There is increasing interest in women's health, including vaginal health. Increasingly, woman are interested in monitoring and feeling in control of their reproductive health, including menstrual health, menstrual cycle tracking, ovulation, and fertility. Further, it has been reported that 75% of women worldwide experience vaginal discharge imbalance, which can cause irritation, inflammation, itchiness, emotional and physical discomfort, and potentially increased risks for pregnant women. It can be challenging for women to identify and obtain help and assistance to address such issues, particularly as it can be challenging to obtain insights into the causes of the imbalance and women may feel reluctant to speak to their healthcare provider about intimate issues.

A problem with current solutions to investigating and monitoring vaginal health is that a woman may require a medical professional to investigate, which some women may not feel comfortable about, and which takes up valuable clinician time. Home care and monitoring solutions may be inaccurate, invasive, challenging or unpleasant to use, and therefore discourage women from feeling capable and empowered to manage their vaginal health.

It is an aim of the present disclosure to address one or more of the disadvantages associated with the prior art.

### SUMMARY OF THE INVENTION

In an aspect there is provided a vaginal device comprising an elongated body comprising an insertion portion at a first end of the body, the insertion portion intended for insertion into a vagina; and a pH sensing device located within the elongated body. The pH sensing device comprises a pH sensor, the pH sensor comprising a glass-free sensing surface located on a surface of the insertion portion, the glass-free sensing surface configured to contact a substance to be pH tested; and a controller connected to the pH sensor, the controller configured to receive pH sensing signalling from the pH sensor and output an indication of a pH of a substance contacted by the glass-free sensing surface.

Advantageously, the device provides an accurate way for a woman to check her vaginal pH and obtain an indication of vaginal health using an easy to use, reusable device. The substance to be pH tested in the vagina may be vaginal discharge, which may be defined as a mixture of liquid, cells, and bacteria that lubricate and protect the vagina. Vaginal health may include a health condition which is a problem to be treated, such as thrush. Vaginal health may include a health condition which is not related to a medical problem but is an insight into the functioning of the woman's body which may be obtained from obtaining the vaginal pH, such as menstrual health, menstrual cycle tracking, ovulation, fertility, or other health or wellbeing condition.

The glass-free sensing surface of the pH sensor helps ensure safe and accurate pH sensing in the sensitive vaginal area. Since the device includes an electronic controller, data obtained through use may be transmitted to an external device, such as the woman's personal electronic device or smartphone, for logging and further processing to obtain insights on vaginal health.

The body of the vaginal device may comprise a resilient inner casing and an elastic outer casing. Advantageously this provides a robust device which can be used repeatedly, and which may be pleasant and comfortable to use. The inner casing may comprise a polymer. Advantageously this may be readily manufactured without undue restriction on the shape or size which may be formed from the polymer. The outer casing may be made from a medical quality material, allowing for safe intra-vaginal use. The outer casing may be waterproof, allowing for safe use inside the vagina as well as allowing the device to be easily cleaned by washing in water or an appropriate cleaning solution. The outer casing may be made from a material of the group consisting of polyurethane, silicone rubber, latex rubber, synthetic rubber, natural rubber, or combinations thereof. Such materials are suitable for devices designed for intimate female use, can be hypoallergenic, easily washed, are available in a wide range of colours, and feel soft and pleasant, to provide a comfortable user experience.

The pH sensor of the vaginal device may comprise an ion-sensitive field effect transistor (FET). As such, the pH sensor may be made with small dimensions, at least compared to the dimensions of a vagina, allowing for accurate pH sensing without undue discomfort for the user by inserting the insertion portion comprising the pH sensor into the vagina.

The glass-free sensing surface of the pH sensor may be coplanar with an outer surface of the insertion portion. "Coplanar" may be understood to relate to both flat and curved surfaces so that the outer surface and glass-free sensing surface are contiguous to form a continuous overall outer surface. This allows for the overall surface of the insertion portion to be smooth allowing for more comfortable use and for easier cleaning.

The pH sensor may be located in an aperture of an outer casing of the body, and an outer peripheral edge of the glass-free sensing surface of the pH sensor may abut the aperture edge and be connected thereto by a watertight seal. Such an arrangement provides a smooth continuous surface overall for more comfortable use and easy cleaning using water or another suitable cleaning solution.

The glass-free sensing surface may comprise PTFE. Advantageously, PTFE is hypoallergenic, flexible, and may be easily cleaned, while providing a suitable outer surface for the pH sensor.

The vaginal device may comprise a handle portion at a second end of the body opposite the first end. The handle portion is intended for location outside the vagina. The controller may be located at least partially in the handle portion. Advantageously the controller may be housed within the handle portion so that the vaginal device is housed within a single handheld unit for comfort and ease of use and ease of cleaning, as well as allowing for greater design choice in producing an aesthetically pleasing product which is pleasant to handle and touch.

The body may comprise a flexible neck portion located between the insertion portion and the handle portion. The pH sensor may be connected to the controller by cabling located at least partially in the flexible neck portion. As such cabling is securely housed within the device and the flexible neck allows for easy and comfortable use of the device.

The handle portion may comprise a wireless charging coil and a rechargeable battery connected to the wireless charging coil, the wireless charging coil configured to operably couple to a wireless charging dock and cause the rechargeable battery to be recharged. As such the device may advantageously be wirelessly used and charged and remain a self-contained device for ease of use and cleaning (i.e. rather than requiring a wired connection for power and/or data transmission).

The elongated body may have a longitudinal direction along the elongated length of the body and a transverse direction perpendicular to the longitudinal direction. The insertion portion may have a length along the longitudinal direction of, for example, less than 5cm. Other examples may have a longer insertion portion. The insertion portion may have a width along the transverse direction of, for example, less than 2cm. Other examples may have a wider insertion portion. The insertion portion may be smaller in the transverse direction than the handle portion in some examples. The neck portion may be smaller in the transverse direction than both the insertion portion and the handle portion in some examples. As such the insertion portion may be formed with small dimensions compared to the vagina and have an ergonomic shape for simple and minimally invasive use. Of course the skilled person will appreciate that a range of dimensions and relative dimensions of the components of the vaginal device may be used and are covered by this disclosure.

The vaginal device may comprise a wireless communications apparatus configured to receive the indication of the pH of the substance contacted by the glass-free sensing surface from the controller and transmit the indication of the pH to an external electronic device. The external electronic device may be, for example, a smartphone, a server, or another personal electronic device. As such, pH data may be recorded remote from the device and stored and processed at the remote device. This allows for the collected data to be processed without restriction on the processing capability needing to be on the device itself. The collected data may in some examples be transmitted to a third party, such as a trusted contact, preferred audience/person such as a partner, spouse, or other family member, or another third party such as a medical or health practitioner.

The vaginal device may comprise a visual indicator configured to output a visual indication of an operation status of the vaginal device. For example, an LED or other indicator may be used to indicate an operational status such as "battery charged"; "battery charging", "battery low", or "sensor error".

The vaginal device may further comprise a temperature sensing device located within the elongated body. The temperature sensing device may comprise a temperature sensor located in the insertion portion and configured to sense a temperature of a substance in contact with the temperature sensor; and a temperature controller connected to the temperature sensor, the temperature controller configured to receive temperature sensing signalling from the temperature sensor and output an indication of a temperature sensed by the temperature sensor. The pH sensor may be configured to sense the pH of a substance and the temperature sensor may be configured to sense the temperature of the substance at the same time. Advantageously both the pH and the temperature of the vagina may be sensed simultaneously to allow for further vaginal health information to be obtained using the single device.

The indication of the pH of the substance contacted by the glass-free sensing surface may be an indication of the pH of vaginal discharge; and the indication of the pH may be provided for processing to provide an indication of vaginal health. The indication of temperature sensed by the temperature sensor may be an indication of the temperature inside the vagina; and the indication of temperature may be provided with the indication of the pH for processing to provide the indication of vaginal health. The indication of vaginal health comprises one or more of an indication of fertility, ovulation, menstrual cycle, and a medical problem. In this way the vaginal device can be used to obtain insights into the woman's health, particularly vaginal and reproductive health.

In an aspect there is provided a method of manufacture of a vaginal device, the method comprising: providing a pH sensor comprising a glass-free sensing surface configured to contact a substance to be pH tested; providing a controller configured to receive pH sensing signalling from the pH sensor and output an indication of a pH of a substance contacted by the glass-free sensing surface; connecting the pH sensor to the controller; providing an elongated body, the elongated body comprising an insertion portion at a first end of the body, the insertion portion intended for insertion into a vagina; and locating the pH sensor and the controller within the body, comprising locating the glass-free sensing surface of the pH sensor on a surface of the insertion portion.

Locating the glass-free sensing surface of the pH sensor on the surface of the insertion portion may comprise: locating the glass-free sensing surface in an aperture of an outer casing of the body with an outer peripheral edge of the glass-free sensing surface of the pH sensor abutting the aperture edge, and connecting the outer peripheral edge of the glass-free sensing surface of the pH sensor to the aperture edge by a watertight seal.

In an aspect there is provided a kit of parts comprising: a cleaning brush; a cleaning fluid; a calibration fluid; and the vaginal device disclosed herein. Advantageously an appropriate cleaning fluid and calibration fluid may be provided with the device for accurate and easy use.

In an aspect there is provided a controller for use in a vaginal device comprising an insertion portion intended for insertion into a vagina and a pH sensor connected to the controller, the pH sensor located in the insertion portion and the pH sensor comprising a glass-free sensing surface located on a surface of the insertion portion and configured to contact a substance to be pH tested; the controller configured to: receive pH sensing signalling from the pH sensor in dependence on contact of the pH sensor with the substance to be pH tested, determine a pH of the substance in dependence on the pH sensing signalling, and output an indication of a pH of the substance to an electronic device. The electronic device may be a display screen of the vaginal device, and/or a remote electronic device.

In some examples the vaginal device may comprise a temperature sensor connected to the controller, the temperature sensor located in the insertion portion and configured to contact a substance to be temperature tested; the controller configured to: receive temperature sensing signalling from the temperature sensor in dependence on contact of the temperature sensor with the substance to be temperature tested, determine a temperature of the substance in dependence on the temperature sensing signalling, and output an indication of a temperature of the substance to an electronic device.

Within the scope of this application it is expressly intended that the various aspects, embodiments, examples and alternatives set out in the preceding paragraphs, in the claims and/or in the following description and drawings, and in particular the individual features thereof, may be taken independently or in any combination. That is, all embodiments and/or features of any embodiment can be combined in any way and/or combination, unless such features are incompatible. The applicant reserves the right to change any originally filed claim or file any new claim accordingly, including the right to amend any originally filed claim to depend from and/or incorporate any feature of any other claim although not originally claimed in that manner.

### BRIEF DESCRIPTION OF THE DRAWINGS

One or more examples will now be described, by way of example only, with reference to the accompanying drawings, in which:
Figure 1 shows a schematic diagram of a vaginal device according to examples disclosed herein;
Figure 2 shows schematically a pH sensor of the vaginal device according to examples disclosed herein;
Figure 3 shows an example of the vaginal device in use according to examples disclosed herein;
Figure 4 shows an system of a vaginal device and a remote electronic device according to examples disclosed herein;
Figure 5 shows schematically a kit of parts according to examples disclosed herein;
Figure 6 shows an example method according to examples disclosed herein; and
Figure 7 shows a schematic processor system of a vaginal device according to examples disclosed herein.

### DETAILED DESCRIPTION

There is increasing interest in women's health, including vaginal health, and there is a need in the field to improve women's health monitoring and to allow women to take control of their personal health. Vaginal health is important for several factors, including gynaecological health, general health, and period and fertility tracking. Currently, if a woman needs to understand more about her vaginal health, often she will need to visit a medical professional to investigate. Further, often home care solutions may be inaccurate, invasive, or challenging to use, and therefore discourage women from feeling capable and empowered to manage their vaginal health.

Examples disclosed here provide a vaginal device for monitoring vaginal health, which includes a pH probe. The device may provide improved ease and comfort of use compared with existing devices, may provide increased accuracy of pH (and also temperature) detection and recording, may be manufactured with an attractive aesthetic, and may interface with a user's personal electronic device for data collection and analysis.

Monitoring vaginal health may be considered to include recording data from sensing pH (and temperature) inside the vagina to obtain insights into a health condition which is a problem to be treated, such as thrush or vaginosis. Monitoring vaginal health may be considered to include recording data from sensing pH (and temperature) inside the vagina to obtain insights into the health of the vagina and of the woman's body which are not necessarily considered a problem to be treated, such as for monitoring menstrual health, menstrual cycle tracking, ovulation, fertility, or other health or wellbeing condition. The composition and properties of vaginal discharge and the temperature inside the vagina can vary according to different states of the woman's body and can give rise to detectable variation in vaginal pH and temperature, such as at different stages through puberty, different phases of the menstrual cycle, during pregnancy, post-partum, and during perimenopause and menopause.

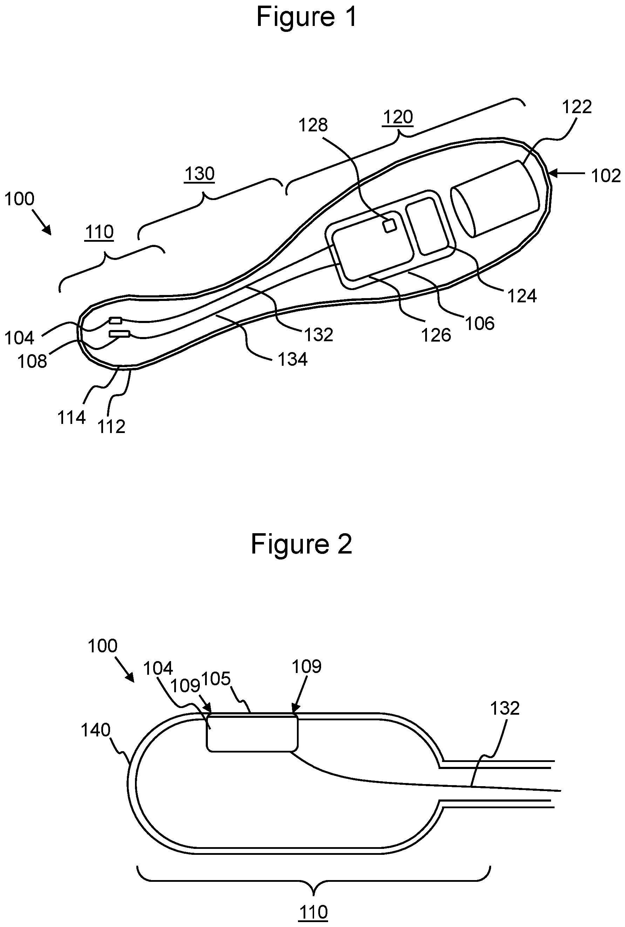

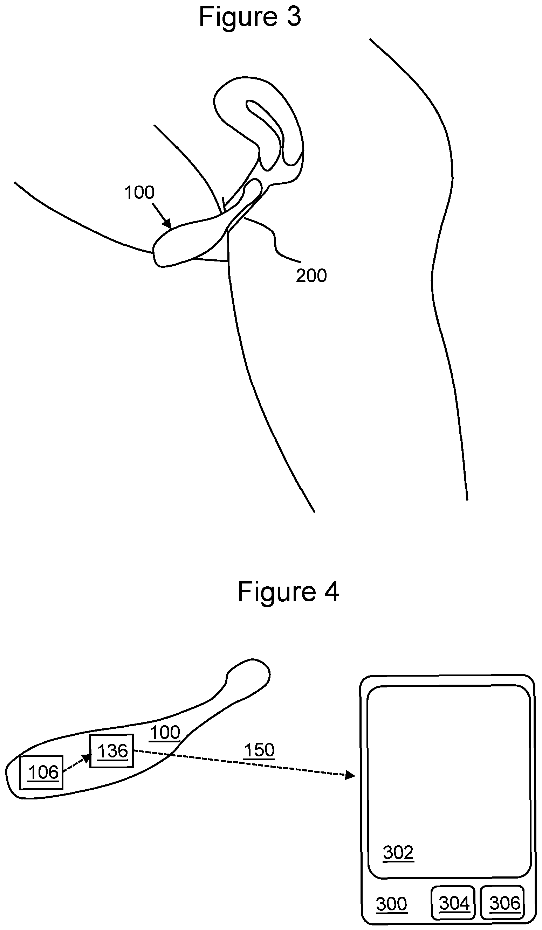

Figure 1 shows schematically a vaginal device 100 comprising an elongated body 102. The elongated body 102 comprises an insertion portion 110 at a first end of the body 102. The insertion portion 102 is intended for insertion into a vagina 200 as shown in Figure 4. The device 100 comprises a pH sensing device 104 located within the elongated body 102. Figure 2 describes the pH sensing device feature in more detail, i.e. that the pH sensing device 104 comprises a pH sensor, and the pH sensor comprises a glass-free sensing surface 105 located on a surface of the insertion portion 110. The glass-free sensing surface 105 is configured to contact a substance to be pH tested, for example, fluid on the vaginal wall.

Figure 1 also shows that the device 100 comprises a controller 106 connected to the pH sensor 104. The controller 106 is configured to receive pH sensing signalling from the pH sensor 104 and output an indication of a pH of a substance contacted by the glass-free sensing surface 105. The substance to be pH tested in the vagina may be vaginal discharge. Vaginal discharge comprises a mixture of liquid (e.g. cervical mucus, vaginal fluid), cells (e.g. shedding vaginal and cervical cells) and bacteria, that lubricates and protects the vagina.

The device 100 can be used to sense the pH in the vagina in an accurate way through the use of the pH sensor. The glass-free sensing surface 105 of the pH sensor helps ensure safe and accurate pH sensing in the sensitive vaginal area. The glass-free sensing surface may comprise material which is resistant to breakage, shatter, chipping and/or splitting, or other damage, particularly during normal use of the vaginal device but also if the device is dropped or knocked, for example. For example, the glass-free sensing surface may be flexible, stretchable, and/or deformable. Generally, at least the insertion portion including the glass-free pH sensor and any other sensor present (e.g. temperature sensor) will be resistant to any breakage, shatter, chipping, splitting, or other damage. The glass-free pH sensor is reusable. Aside from the glass-free pH sensor, as will be described, other advantageous features may be incorporated in the device 100 to provide an accurate way for a woman to check her vaginal health and pH using an easy to use, reusable device. For example, at least the insertion portion outer surface which is to contact the vagina wall may be hypoallergenic and/or may have a smooth surface texture for ease of use.

Since the device 100 includes an electronic controller 106, data obtained through use may be transmitted to an external device, such as the woman's personal electronic device or smartphone, for logging and further processing to obtain insights on vaginal health. This is described in more detail with reference to Figure 4.

Figure 1 also shows that the body 102 of the vaginal device 100 may comprise a resilient inner casing 114 and an elastic outer casing 112. Advantageously this provides a robust device 100 which can be used repeatedly by way of the resilient inner casing 114 providing structure to the device 100 and protection for the components housed within the casing. "Resilient" should be understood to mean that at least part of the casing may be elastically deformable to some degree (e.g. the flexible neck portion 130) but the outer casing 112 causes the device 100 to retain a defined shape when no forced are applied thereto. The inner casing 112 may comprise a polymer such as a plastic. Advantageously this may be readily manufactured without undue restriction on the shape or size which may be formed.

Further, the elastic outer casing 114 also can be elastically deformed, and allows for materials to be used on the outer surface of the device 100 which may be pleasant and comfortable to use. The outer casing 114 may be made from a medical quality material, allowing for safe intra-vaginal use. The outer casing may be waterproof 114, allowing for safe use inside the vagina as well as allowing the device 100 to be easily cleaned by washing in water or an appropriate cleaning solution. The outer casing 114 may be made from a material of the group consisting of polyurethane, silicone rubber, latex rubber, synthetic rubber, natural rubber, or combinations thereof. Such materials are suitable for devices designed for intimate female use, can be hypoallergenic, easily washed, are available in a wide range of colours, and feel soft and pleasant, to provide a comfortable user experience.

The vaginal device 100 may comprise a handle portion 120 at a second end of the body 102 opposite the first end. The handle portion 120 is intended for location outside the vagina. The device 100 may be easily handled so that the user can hold the handle portion 120 to guide the insertion portion 110 of the device 100 inside the vagina. The handle portion 120 may be dimensions for a comfortable fit in a woman's hand and may be proportioned such that it is clear that the handle is not intended for insertion into the vagina.

In some examples the controller 106 may be located at least partially in the handle portion 120. Advantageously the controller 106 may be housed within the handle portion 120 so that the vaginal device 100 is housed within a single self-contained handheld unit for comfort and ease of use and ease of cleaning, as well as allowing for greater design choice in producing an aesthetically pleasing product which is pleasant to handle and touch (i.e. there is no need for an external cable connected to an external control system, for example).

The body 102 may comprise a flexible neck portion 130 located between the insertion portion 110 and the handle portion 120. The pH sensor 104 may be connected to the controller 106 by cabling 132 located at least partially in the flexible neck portion 130. As such, cabling (which is flexible) is securely housed within the device 100 and located in the flexible neck so the cabling does not hinder the flexibility of the neck portion 130.

The handle portion 120 may comprise a wireless charging coil 122 and a rechargeable battery 124 connected to the wireless charging coil 122. The wireless charging coil 122 may be configured to operably couple to an external wireless charging dock and cause the rechargeable battery 124 to be recharged. As such the device 100 may advantageously be wirelessly used and charged, and remain as a self-contained device 100 for ease of use and cleaning. Users are likely to be familiar with recharging the power of a portable electronic device by locating the device on a charging station or docking station.

The elongated body 102 may have a longitudinal direction along the elongated length of the body and a transverse direction perpendicular to the longitudinal direction. The insertion portion 110 may have a length along the longitudinal direction of less than 5cm. The insertion portion 110 may have a width along the transverse direction of less than 2cm. The insertion portion 110 may be smaller in the transverse direction than the handle portion 120. The neck portion 130 may be smaller in the transverse direction than both the insertion portion 110 and the handle portion 130. As such the insertion portion 110 may be formed with small dimensions compared to the vagina (and the handle) and the device may have both an ergonomic shape which fits the hand easily, and a small insertion portion 110 for minimally invasive use.

It will be appreciated that the schematic illustration of Figure 1 should not be understood as limiting the shape or relative dimensions of the device 100. Other possible shapes include a device 100 having a curve in the neck portion 130 which may facilitate easy insertion for some users, or a shape having an ergonomic grip form at the handle portion 120 to assist the user to hold the device in an appropriate way for ease of insertion (e.g. by guiding the user's fingers and thumb to hold the device in a manner facilitating insertion).

The vaginal device 100 may, as shown, comprise a wireless communications apparatus 126 configured to receive the indication of the pH of the substance contacted by the glass-free sensing surface 105 from the controller 106 and transmit the indication of the pH to an external electronic device. This is described in more detail in relation to Figure 4.

The vaginal device 100 may comprise a visual indicator 128 configured to output a visual indication of an operation status of the vaginal device 100. For example, an LED or other indicator 128 may be used to indicate, by way of displaying a particular colour or flashing pattern for example, an operational status such as "battery charged"; "battery charging", "battery low", or "sensor error". The visual indicator 128 in some examples may comprise a display screen configured to indicate some operational status message, use instructions, sensor reading results, or a personalised message for the user, for example. The potential uses of such a display screen will be evident to the skilled person.

The vaginal device 100 may, in some examples, further comprise a temperature sensing device 108 located within the elongated body 102. The temperature sensing device 108 may comprise a temperature sensor located in the insertion portion 110 and may be configured to sense a temperature of a substance in contact with the temperature sensor 108. A temperature controller 106 may be connected to the temperature sensor 108 (in this example the pH controller and temperature controller are the same element 106). The temperature controller 106 may be configured to receive temperature sensing signalling from the temperature sensor 108 and output an indication of a temperature sensed by the temperature sensor. The pH sensor 104 may be configured to sense the pH of a substance and the temperature sensor 108 may be configured to sense the temperature of the substance at the same time in some examples. Advantageously, both the pH and the temperature of the vagina may be sensed simultaneously to allow for further vaginal health information to be obtained using the single device 100 and for correlations to be made between the pH and temperature characteristics because they are measured at the same time.

In some examples, both the pH and temperature may be sensed but the user may choose only to record one of those sensed parameters. It may be advantageous to be able to record both pH and temperature simultaneously so that any trends or correlations may be determined when the collected data is later processed.

For example, a woman who would like to understand her fertility may use the vaginal device to monitor her vaginal temperature and pH. For example, determining that the woman has a basic vaginal pH and low temperature may indicate ovulation, an acidic vaginal pH and temperature below a predetermined threshold may indicate pre ovulation phase, and an acidic vaginal pH and temperature above a predetermined threshold may indicate post-ovulation / luteal phase. As another example, a woman may wish to monitor her vaginal pH to better understand how that correlates with a medical issue and potentially obtain an insight in how to avoid or mitigate against the occurrence of the issue. For example, an onset of vaginal thrush, low mood, vaginal dryness, or other condition may correlate with vaginal pH and the ability to track vaginal pH may be used to aid in preventing or reducing the issue by, for example, changing hygiene routine, diet, stress factors, or other factors. The vaginal device may be used daily to regularly track vaginal pH and temperature, for example as part of a woman's normal daily routine. In other examples it may be used more than daily, or less frequently, though the information which can be determined about vaginal health may not necessarily be improved by more than daily use, and may be less accurate with less than daily frequency use.

Figure 2 shows schematically a pH sensor 104 of the vaginal device 100. The pH sensor 104 comprises a glass-free sensing surface 105. The glass-free sensing surface 105 of the pH sensor 104 may be coplanar with an outer surface 140 of the insertion portion 110. "Coplanar" may be understood to relate to both flat and curved surfaces; a flat surface is illustrated but in other examples the surface may be concave, convex, or some other non-planar form. A sensing surface 105 which is coplanar with the outer surface of the insertion portion 110 may allow for the overall surface of the insertion portion 110 (plus sensing surface 105) to be smooth and continuous, allowing for more comfortable use and for easier cleaning.

The pH sensor 104 of the vaginal device 110 may comprise an ion-sensitive field effect transistor (FET). An ion-sensitive FET may advantageously be used as it can be used to sense pH in a range of fluids including low viscosity fluid, "semi" fluids of low to medium viscosity, and pastes of medium to high viscosity, thereby being well suited to the vagina which may contain fluid(s) having a range of viscosities. The pH sensor 104 itself may comprise an elongated portion of the order of 100mm-150mm in length (which may be located within the insertion portion 100 and the neck portion 130) and a tip portion where the glass-free sensing surface is located at one end of the elongated portion of the order of approximately 1 mm to 5mm in length. As such, the pH sensor, in particular the glass-free sensing surface 105, may be made with small dimensions, at least compared to the dimensions of a vagina, allowing for incorporation into the device 100 and accurate pH sensing without undue discomfort for the user due to having a bulky or size or shape unsuitable for comfortable vaginal insertion.

The pH sensor may be located in an aperture of an outer casing of the body (i.e. of the insertion portion 110) as shown in Figure 2. An outer peripheral edge 109 of the glass-free sensing surface 105 of the pH sensor may abut the aperture edge, and be connected thereto by a watertight seal. Such an arrangement provides a smooth continuous surface of the insertion portion 110 overall, allowing for comfortable use and easy cleaning. Further, by having a continuous form without small grooves or creases where two components are joined reduces the possibility of bacteria or other organisms undesirably remaining in the grooves/creases which may be difficult to thoroughly clean.

The glass-free sensing surface 105 may comprise PTFE. Advantageously, PTFE is hypoallergenic, flexible, and may be easily cleaned, while providing a suitable outer surface for the pH sensor to operate. Other parts of the pH sensor device, such as a housing of the sensor within the inner casing 114 may be formed of any suitable material such as acrylonitrile butadiene styrene (ABS).

Figure 3 shows an example of the vaginal device in use. The insertion portion 110 of the device 100 is inserted into the vagina 200 and may be able to record a pH reading in a few seconds (for example, up to 5 seconds). In some examples the device 100 may comprise a visual or tactile marking on the outer surface 112 which serves to indicate a delineation between the portion of the device 100 (comprising the insertion portion 110) intended to be inserted into the vagina and the portion of the device 100 (comprising the handle portion 120) intended to remain outside the vagina when the device 100 is in use. Such a marking may aid use by allowing the user to easily and confidently understand how to use the device and ensure the pH sensing surface 105 is located at an appropriate position inside the vagina for pH sensing without unnecessary insertion.

Figure 4 shows an system of a vaginal device 100 comprising a controller 106, and a remote electronic device 300. The vaginal device 100 in some examples may comprise a wireless communications apparatus 136 which is configured to receive the indication of the pH of the substance contacted by the glass-free sensing surface 105 from the controller 106 and transmit the indication of the pH 150 to an external electronic device 300. The remote electronic device 300 may comprise a processor 304 and a memory 306. The remote device 300 may comprise an output element 302, such as a display screen or touch screen display, configured to present come information relating to the received pH indication 150.

The controller 106 may be configured to receive pH sensing signalling from the pH sensor in dependence on contact of the pH sensor with the substance to be pH tested, determine a pH of the substance in dependence on the pH sensing signalling, and output an indication of a pH of the substance 150 to an electronic device 300. The vaginal device may comprise a temperature sensor connected to the controller. The controller 106 in such examples may be configured to: receive temperature sensing signalling from the temperature sensor in dependence on contact of the temperature sensor with the substance to be temperature tested, determine a temperature of the substance in dependence on the temperature sensing signalling, and output an indication of a temperature 150 of the substance to an electronic device. The electronic device 300 may be a remote electronic device 300 as shown in some examples, and the indication of the pH 150 is output by wireless transmission. In some examples there may be a display screen on the vaginal device (e.g. in the handle portion 120) in some examples which can visually indicate the pH and/or temperature so the user can easily see the detected pH and temperature values at the point of performing the check without necessarily referring to the remote device 300.

The external electronic device 300 may be, for example, a smartphone, a laptop computer, a desktop computer, a tablet computer, another personal electronic device, or a server. As such, pH data (and temperature data if collected) may be recorded remotely from the vaginal device 100 and stored and processed at the remote device 300. This allows for the collected data to be processed without restriction on the processing capability needing to be on the device 100 itself. In some examples the electronic device 300 may run software configured to process the received pH data (and temperature data if received). The device may then provide an indication for the user, for example relating to vaginal health, ovulation, fertility, menstrual cycle, general health, or other health and wellbeing status which depends, at least in part, on vaginal pH information (and temperature information if collected), and possibly other information such as the user's demographic data, or user inputs to indicate their health, wellbeing, diet, activity levels, or other relevant factors. In some examples the collected data, may for example, be transmitted to third party such as a medical practitioner or women's care practitioner. It will be appreciated that various data analysis methods are available, such as comparison with a pre-stored data set of known correlations between vaginal pH and temperature and health conditions, for example, for obtaining the health information from the sensor data which the device may employ.

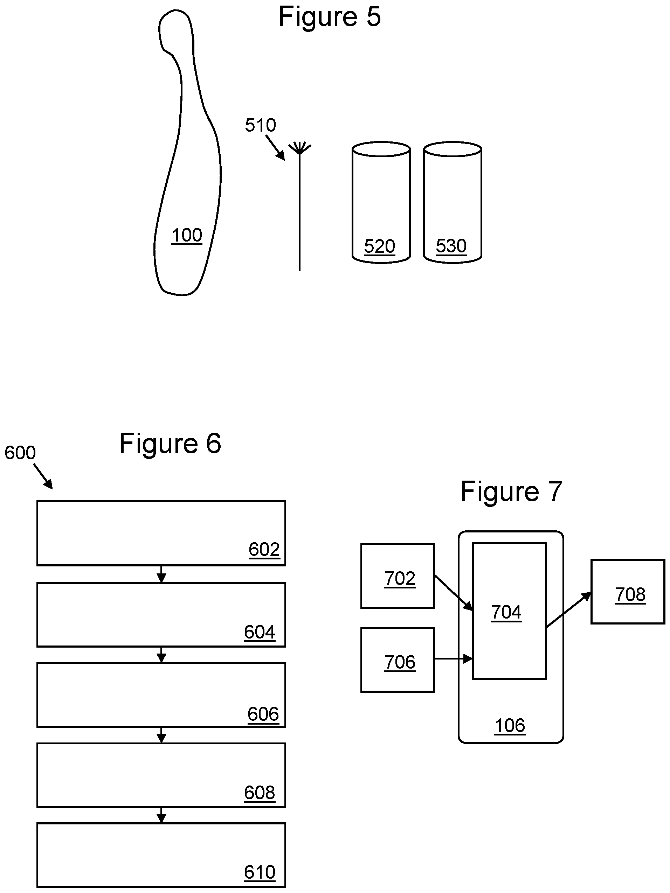

Figure 5 shows schematically a kit of parts. It may be advantageous to provide a kit of suitable components to be used together with the vaginal device. The kit of parts illustrated here comprises a cleaning brush 510 suitable for cleaning the vaginal device; a cleaning fluid 520 (for example, distilled water or other fluid suitable for cleaning items designed for contacting intimate body parts); a calibration fluid 530 (such as a pH buffer solution or pH buffer solution set of solutions of different pHs (e.g. pH 4, pH 7 and pH 10)); and the vaginal device 100 disclosed herein.

Figure 6 shows an example method 600 of manufacture of a vaginal device as disclosed herein. The method 600 comprises: providing a pH sensor comprising a glass-free sensing surface configured to contact a substance to be pH tested 602; providing a controller configured to receive pH sensing signalling from the pH sensor and output an indication of a pH of a substance contacted by the glass-free sensing surface 604; connecting the pH sensor to the controller 606; providing an elongated body, the elongated body comprising an insertion portion at a first end of the body, the insertion portion intended for insertion into a vagina 608; and locating the pH sensor and the controller within the body, comprising locating the glass-free sensing surface of the pH sensor on a surface of the insertion portion 610. Locating the glass-free sensing surface of the pH sensor on the surface of the insertion portion 610 may comprise: locating the glass-free sensing surface in an aperture of an outer casing of the body with an outer peripheral edge of the glass-free sensing surface of the pH sensor abutting the aperture edge, and connecting the outer peripheral edge of the glass-free sensing surface of the pH sensor to the aperture edge by a watertight seal. Thus a vaginal device as disclosed herein may be manufactured.

The blocks illustrated in Figure 6 represent steps in a method 600 but it will be appreciated that the illustration of a particular order to the blocks does not necessarily imply that there is a required or preferred order for the blocks and the order and arrangement of the block may be varied. Furthermore, it may be possible for some steps to be omitted or added in other examples.

Figure 7 shows a schematic processor 704 of a vaginal device 100 according to some examples. The processor 704 may be part of the controller 106. The processor 704 is configured to receive the pH sensing signalling 702 from the pH sensor 104 of the vaginal device 100. In this example the processor 704 is also configured to receive temperature sensing signalling 706 from the temperature sensor. The processor 704 is configured to output an indicator 708 of the sensed pH and an indicator 708 of the sensed temperature. The indicator 708 may be provided to an external device 300 for logging and/or processing as discussed in relation to Figure 4.

As used here, 'connected' means either 'mechanically connected' or `electrically connected' either directly or indirectly. Connection does not have to be galvanic. Where the controller is concerned, connected means operably coupled to the extent that messages are transmitted and received via the appropriate communication means. The term "controller" may be understood to cover a controller, control module, or control element and need not necessary be a multi-element or distributed system (although it may be in some examples). Through this disclosure the term "woman" is intended to mean a person with a vagina which may include people other than cisgender women.

It will be appreciated that various changes and modifications can be made to the present disclosed examples without departing from the scope of the present application as defined by the appended claims. Whilst endeavouring in the foregoing specification to draw attention to those features believed to be of particular importance it should be understood that the Applicant claims protection in respect of any patentable feature or combination of features hereinbefore referred to and/or shown in the drawings whether or not particular emphasis has been placed thereon.

## Claims

1. A vaginal device comprising:
an elongated body comprising an insertion portion at a first end of the body, the insertion portion intended for insertion into a vagina; and
a pH sensing device located within the elongated body, the pH sensing device comprising:
a pH sensor, the pH sensor comprising a glass-free sensing surface located on a surface of the insertion portion, the glass-free sensing surface configured to contact a substance to be pH tested; and
a controller connected to the pH sensor, the controller configured to receive pH sensing signalling from the pH sensor and output an indication of a pH of a substance contacted by the glass-free sensing surface.

2. The vaginal device of claim 1, wherein the body comprises:
a resilient inner casing; and
an elastic outer casing.

3. The vaginal device of any preceding claim, wherein one or more of:
the pH sensor comprises an ion-sensitive field effect transistor; and
the pH sensor is located in an aperture of an outer casing of the body, and an outer peripheral edge of the glass-free sensing surface of the pH sensor abuts the aperture edge and is connected thereto by a watertight seal.

4. The vaginal device of any preceding claim, wherein one or more of:
the glass-free sensing surface is coplanar with an outer surface of the insertion portion; and
the glass-free sensing surface comprises Polytetrafluoroethylene, PTFE.

5. The vaginal device of any preceding claim, comprising a handle portion at a second end of the body opposite the first end, the handle portion intended for location outside the vagina, wherein the controller is located at least partially in the handle portion;
optionally, wherein the body comprises a flexible neck portion located between the insertion portion and the handle portion; and
further optionally, wherein the pH sensor is connected to the controller by cabling located at least partially in the flexible neck portion.

6. The vaginal device of claim 5, wherein the handle portion comprises a wireless charging coil and a rechargeable battery connected to the wireless charging coil, the wireless charging coil configured to operably couple to a wireless charging dock and cause the rechargeable battery to be recharged.

7. The vaginal device of any preceding claim, wherein the elongated body has a longitudinal direction along the elongated length of the body and a transverse direction perpendicular to the longitudinal direction, and one or more of:
the insertion portion has a length along the longitudinal direction of less than 5cm;
the insertion portion has a width along the transverse direction of less than 2cm.

8. The vaginal device of claim 7 when dependent on claim 5, wherein the insertion portion is smaller in the transverse direction than the handle portion.

9. The vaginal device of claim 7 when dependent on claim 5 wherein the body comprises a flexible neck portion located between the insertion portion and the handle portion, wherein the neck portion is smaller in the transverse direction than both the insertion portion and the handle portion.

10. The vaginal device of any preceding claim, comprising a wireless communications apparatus configured to receive the indication of the pH of the substance contacted by the glass-free sensing surface from the controller and transmit the indication of the pH to an external electronic device.

11. The vaginal device of any preceding claim, comprising a visual indicator configured to output a visual indication of an operation status of the vaginal device.

12. The vaginal device of any preceding claim, further comprising a temperature sensing device located within the elongated body, the temperature sensing device comprising:
a temperature sensor located in the insertion portion and configured to sense a temperature of a substance in contact with the temperature sensor; and
a temperature controller connected to the temperature sensor, the temperature controller configured to receive temperature sensing signalling from the temperature sensor and output an indication of a temperature sensed by the temperature sensor;
optionally, wherein the pH sensor is configured to sense the pH of a substance and the temperature sensor is configured to sense the temperature of the substance at the same time.

13. The vaginal device of any preceding claim, wherein:
the indication of the pH of the substance contacted by the glass-free sensing surface is an indication of the pH of vaginal discharge; and
the indication of the pH is provided for processing to provide an indication of vaginal health;
optionally, wherein:
the indication of temperature sensed by the temperature sensor is an indication of the temperature inside the vagina; and
the indication of temperature is provided with the indication of the pH for processing to provide the indication of vaginal health;
further optionally, wherein the indication of vaginal health comprises one or more of an indication of fertility, ovulation, menstrual cycle, and a medical problem.

14. A method of manufacture of a vaginal device, the method comprising:
providing a pH sensor comprising a glass-free sensing surface configured to contact a substance to be pH tested;
providing a controller configured to receive pH sensing signalling from the pH sensor and output an indication of a pH of a substance contacted by the glass-free sensing surface;
connecting the pH sensor to the controller;
providing an elongated body, the elongated body comprising an insertion portion at a first end of the body, the insertion portion intended for insertion into a vagina; and
locating the pH sensor and the controller within the body, comprising locating the glass-free sensing surface of the pH sensor on a surface of the insertion portion;
optionally, wherein locating the glass-free sensing surface of the pH sensor on the surface of the insertion portion comprises:
locating the glass-free sensing surface in an aperture of an outer casing of the body with an outer peripheral edge of the glass-free sensing surface of the pH sensor abutting the aperture edge, and
connecting the outer peripheral edge of the glass-free sensing surface of the pH sensor to the aperture edge by a watertight seal.

15. A kit of parts comprising:
a cleaning brush;
a cleaning fluid;
a calibration fluid; and
the vaginal device of any of claims 1 to 13.
